# EUROPEAN PATENT APPLICATION

(11) **EP 1 764 067 A1**
(43) Date of publication of application: **21.03.2007**
(21) Application number: 05108566.0
(22) Date of filing: 16.09.2005
(51) Int. Cl.: A61F 13/15, B65D 33/28, B65D 85/16, B31B 19/90, B31B 19/26

(54) **Reclosable flexible package for absorbent articles**

(71) Applicant: THE PROCTER & GAMBLE COMPANY, Cincinnati, Ohio 45202 (US)
(72) Inventor: Ulas, Ibrahim, 61440, Oberursel (DE)
(74) Representative: Kremer, Véronique Marie Joséphine

(57) **Abstract**

The present invention relates to a package (10) for containing and dispensing absorbent articles. Specifically, the present invention teaches a flexible pouch-like container enclosing absorbent articles. The container is provided with a drawing means (50), which can be pulled for closing the package. The drawing means is located inside a channel (42) and has two free ends, which can be grasped by the user. Pulling the free ends closes the upper end of the container. Further, a process for making such a package is disclosed.

## Description

### FIELD OF THE INVENTION

The present invention relates to a package for containing and dispensing absorbent articles. Specifically, the present invention teaches a flexible pouch-like container enclosing absorbent articles. The container is provided with a drawing means, which can be pulled for closing the package. The drawing means is located inside a channel and has two free ends, which can be grasped by the user. Pulling the free ends closes the upper end of the container. Further, a process for making such a package is disclosed.

### BACKGROUND OF THE INVENTION

Absorbent articles for personal hygiene, such as feminine care articles like sanitary napkins, tampons or panty liners, are marketed in manifold form by many different suppliers. Usually, such articles are sold to the consumer as stacks of such articles packaged in either carton boxes or pouch bags made of polymeric film. Although such packages provide hygienic storage to the contained articles during shelf live, once opened, these packages cannot be closed completely anymore and thus allow access of contaminants and bacteria into the package and thus to the stack of absorbent articles.

Therefore, a continuing need exists for a package for absorbent articles, which provides hygienic protection to the articles contained therein even after having been opened by the consumer. Ease of handling of such a package would be another key requirement.

### SUMMARY OF THE INVENTION

The present invention encompasses a package for absorbent articles. The package comprises a container having an interior space. A plurality of absorbent articles is disposed within the interior space. The container has a bottom end and a top end. At or near the top end of the container a frangible closure is arranged such that the absorbent articles are enclosed in the interior space of the container between the bottom end and the frangible closure. The top end is provided with a drawing means, preferably a drawing ribbon or draw string, partially enclosed in a channel located at or near the top end of the container. The drawing means has two free ends extending out of the channel. The free ends are arranged graspable for the user, such that by pulling those free ends the channel and thereby the top end of the container is gathered together, which closes the top end of the container. Further, the free ends extend out of the channel at adjacent locations, such that both free ends can be grasped with the same hand by the user. This is a clear handling benefit as the container can then be held with the other hand.

### BRIEF DESCRIPTION OF THE DRAWINGS

- Fig. 1: illustrates a preferred embodiment of the package of the present invention in perspective view.
- Fig. 2: illustrates a top view of the package shown in Fig. 1.
- Fig. 3: illustrates the package shown in Fig. 1 in closed state when the user has pulled on the free ends of the drawing means such that the top end is gathered together.
- Fig. 4: is a scheme illustrating the process of the present invention for making containers.
- Fig. 5: is a cross-sectional view of Fig. 4 along the line 5-5.

### DETAILED DESCRIPTION OF THE INVENTION

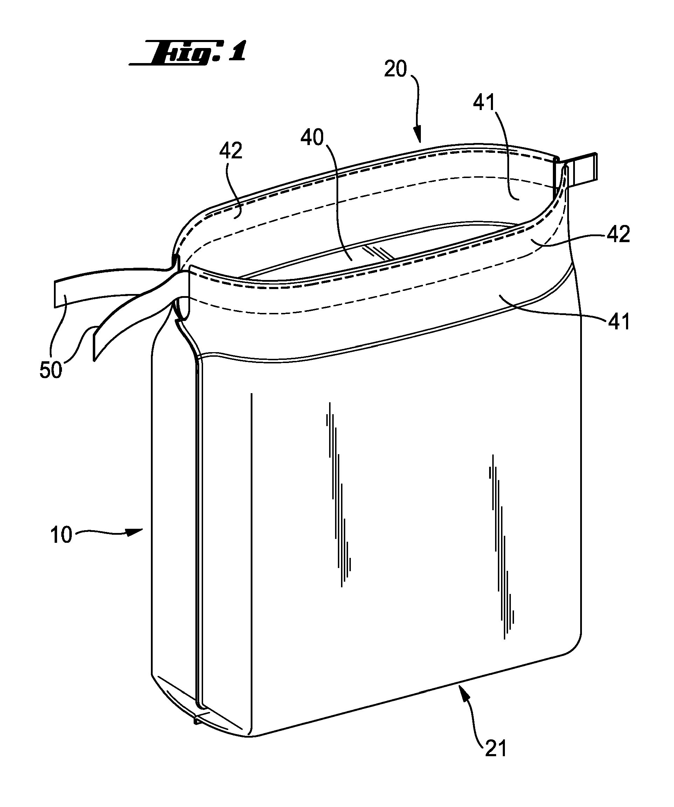

Referring to FIG. 1, one embodiment of the package of the present invention is shown. The flexible container 10 is made from polymer package material. The container has a top end 20 and a bottom end 21. The container may be any shape known in the art. For example, the container may have a polyhedral shape defining or forming a polyhedral enclosure. The articles disposed within the interior space may all be identical to one another or may be different from one another. The articles are preferably disposable absorbent articles, such as flat or folded sanitary napkins or panty liners in a stacked configuration.

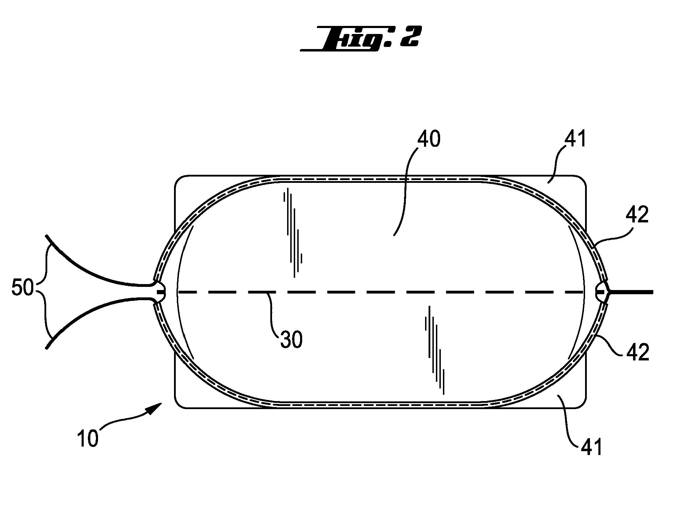

The container 10 is closed for shipping and marketing, and has a frangible closure 30 for opening or otherwise accessing the interior space for easy access to the articles. As shown in FIG. 2, the frangible closure 30 can be a line of weakness, such as a line of perforations. Such lines can have any configurations/shapes, e.g. straight or curved. In alternative embodiments, the frangible closure 30 for accessing the interior space can be formed of an opening in the material of the container, which is releasably closed by an adhesive tab.

The package material may be composed of different materials or may be composed of substantially the same type of material. The package material may be composed of a laminate. The package material can be any flexible material. The package material can be a blown or cast film of polyethylene, or a blend of low density polyethylene and linear low density polyethylene, or other polymers suitable for flexible films, including metallocene, ethylene vinyl acetate, surlyn, polyethylene terephtalate, biaxially oriented polypropylene, and/or nylon. The package material can be cloth. The package material can be a nonwoven material. The package material can be a woven material. Other materials suitable for the package material are metallised polymeric films.

The absorbent articles contained in the package of the present invention can be of any type known in the art. The absorbent articles preferably are disposable absorbent articles such as diapers, catamenial devices, tampons, sanitary napkins, pantiliners, or adult incontinence products. The articles might also be tissues, wipes and other hygienic absorbent articles.

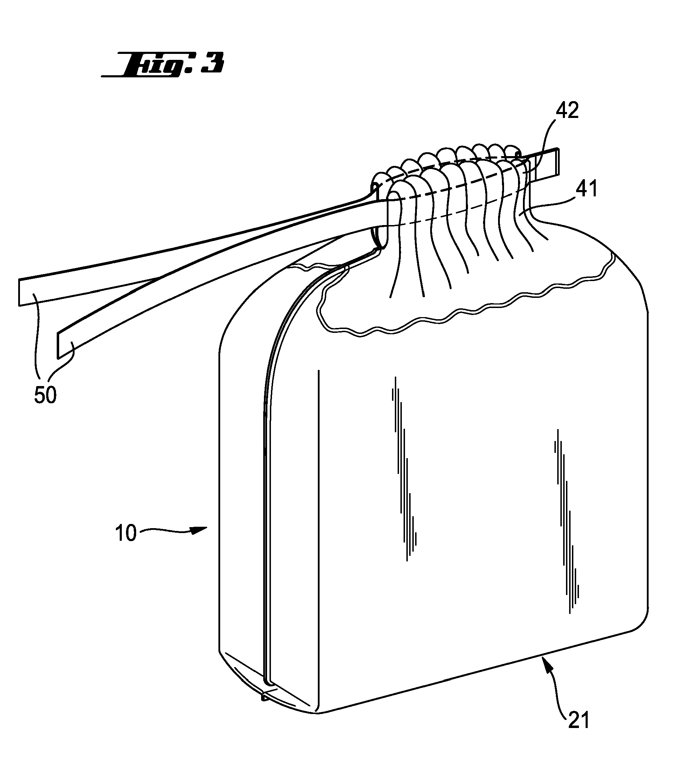

One preferred embodiment of the present invention is shown in FIG. 1. The top end 20 of the container 10 has a top panel 40 and extensions 41, the extensions 41 extending above top panel 40 when container is oriented as shown in FIG. 1. Side extensions 41 comprise a channel 42 formed by folding over the package material in the extension area. A seam, such as a thermally-sealed line can seal the channel 42. Inside channel 42 the container comprises a drawing means such as a drawing ribbon 50. The two fee ends of the drawing ribbon 50 extend out of the channel 42. The drawing means is disposed to move freely inside channel 42 such that the top end 20 of container 10 can be drawn closed as shown in FIG. 3. By this, the extensions 41 are gathered together.

In use, the user can unseal the package by breaking the frangible closure 30, which can be seen in Figure 2. The frangible closure 30 can be constituted of a line of weakness or line of perforations, a tear tape or an adhesive flap or piece of adhesive tape, which covers an opening. After unsealing the container 10 by opening the frangible closure 30 the user can remove an article from inside the interior of the container 10, and then re-close the container by pulling on the drawing means 50 in an easy and convenient manner. Once closed, the drawing means 50 can be used as a convenient carrying handle.

In one embodiment herein the drawing means 50 is a drawing ribbon. The drawing ribbon 50 can be pulled, thereby gathering in the extensions 41 top end 20, as illustrated in Figure 3. When the drawing ribbon 50 is pulled to its substantially maximum amount, the top panel 40 of the container 10 can be closed such that the articles contained within cannot fall out. The drawing ribbon 50 in a pulled position is an easy and convenient means for the consumer to carry the container 10.

The drawing means 50 can be made of any thin flexible material. Exemplary drawing means are made of blown or cast film in a blend of low density polyethylene and linear low density polyethylene, metallocenes, ethylene vinyl acetate, Surlyn^{®}, polyethylene terephtalate, biaxially oriented polypropylene, and/or nylon. Other suitable materials for the drawing means 50 herein are cloth, nonwoven or woven materials.

The drawing means 50 is disposed to move freely inside the channel 42. The drawing means 50 has two free ends, which extend out of the channel 42 at adjacent locations. 'Adjacent locations' herein means that the distance between the locations, where the individual free ends are extending out of the channel 42, is not more than 5 cm, preferably 2 cm, more preferably 1cm and most preferably 0.5 cm. The free ends are arranged graspable for the user. By pulling the free ends the user can close the top end of the container 10. This allows a hygienically satisfactory storage of the remaining articles stored in the container 10 after the container 10 has been opened as the closed top end provides protection from contamination by dust, moisture and the like.

In an alternative embodiment the free ends of the drawing means 50 are joined by a readily frangible joint for preventing the drawing means 50 from being pulled out of the channel unintentionally during transport and shelve life. This frangible joint easily breaks when the user pulls the two free ends.

An additional advantage of this particular arrangement of the free ends is that the user can pull both free ends with one hand at a time and securely hold the package with his other hand therewhile. This represents a clear advantage over executions known from the prior art, where free ends of drawings strings extend out of the channel in opposite locations of the upper end of the container. When pulling on these strings the user cannot hold the package itself but has to pull on one free end with each of his hands. The inability to hold the container when pulling on the drawing strings gives rise to certain disadvantages, such as articles may fall out of the not yet closed container.

To permit optimized stacking, storing, packing, or shelving, the container 10 can be modified by folding down the side extensions 41. By this the side extensions 41 lie substantially flat against sides of the container 10. Such a configuration allows more stable stacking and shelving by requiring less space. When the container is used, i.e. when absorbent articles are dispensed and the frangible closure is opened, the side extensions can be folded back into their upright position for being able to fulfil their functionality.

Container 10 can be made by methods known in the art, such as by known "flow wrap" processes in which a web of material is conveyed, cut, folded, seamed, filled and closed. Flow wrap processes can produce generally "cube-shape" or polyhedral-shaped containers. Alternatively, the container can be formed out of pre-made bags, formed on or off the packing line. These pre-made bags can be provided in many way, such as individually or in the form of a stack (wicket), which is fed into the packing line. Such pre-made bags are opened, filled and sealed.

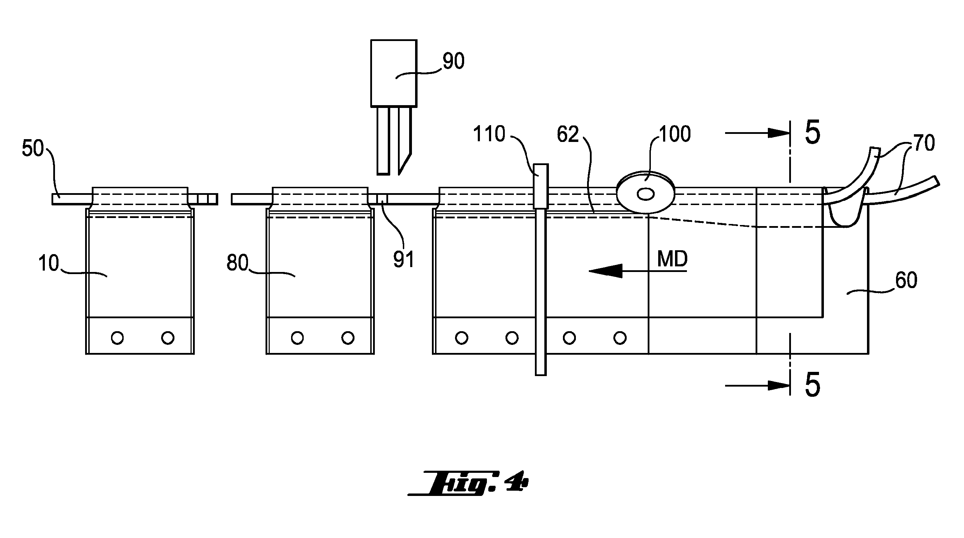

In the following a preferred process for producing the container according to the present invention will be described, as illustrated inn Figure 4. The process is continuous. It can be structured into four steps:
- supplying and folding the continuous package material 60,
- supplying string-like material 70 and forming the channels enclosing said string-like material 70,
- cut-sealing the continuous package material 60 in cross direction, whereby forming individual compartments 80, and
- forming the drawing means by feeding the individual compartments 80 through a cut & join unit 90.

'Machine direction' as used herein refers to the direction of the production line. 'Cross-direction' as used herein refers to a direction, which extends perpendicularly to said machine direction.

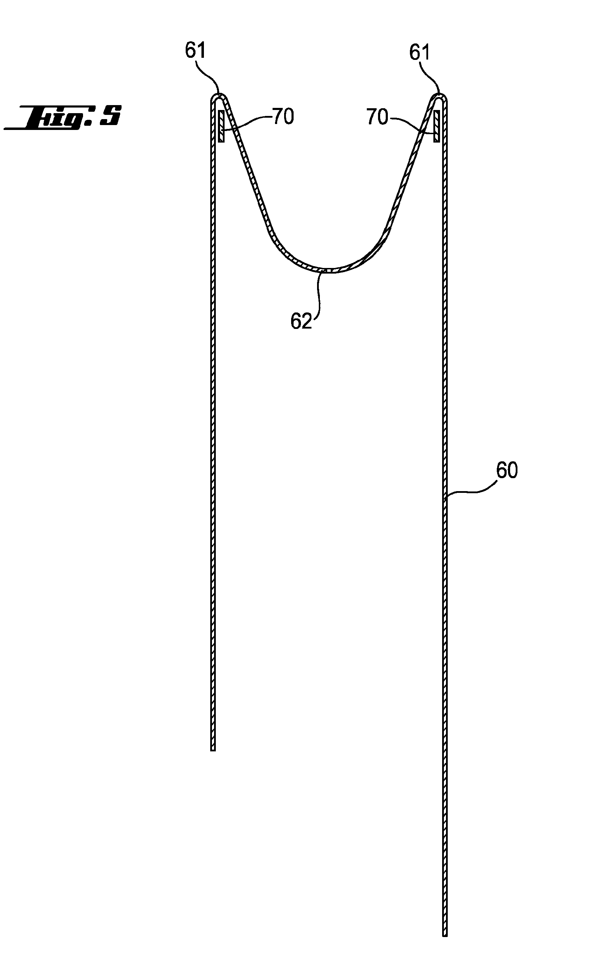

In the first stage of the process a continuous package material 60 is supplied in a machine direction MD. This continuous package material 60 is then folded such that it assumes an M-shaped cross-section in cross direction. Figure 5 illustrates the M-shaped cross-section, as present in region 6-6. The M-shape is characterized by two tips 61 in the outer fold lines and one inner fold line 62.

In the second step the continuous M-shaped package material 60 is provided with continuous string-like material 70, which is fed into each of the two tips 61 of the continuous package material 60. This string-like material 70 will form the drawing means in the fmal container. The continuous string-like material 70 is thermally incompatible with the continuous package material 60, i.e. it has a higher melting point. 'Thermally incompatible' as used herein means that the melting point difference between two materials is at least 100°C, preferably more than 100°C. In a next step the continuous package material 60 will be sealed near each tip 61 by a sealing line 62 in machine direction, thereby forming a channel enclosing the continuous string-like material 70 between each of the tips 61 and the sealing lines 62. A suitable means for forming the sealing lines 62 are heated rollers 100, which heat seal the continuous package material 60 linearly.

In the next step individual compartments 80 are formed. The continuous package material 60 with the channels enclosing the continuous string-like material 70 will be fed through a hat cutting section 100, where the continuous package material 60 is cut in cross-direction by heated blades, which are heated to the melting point of the continuous package material 60 but below the melting point of the continuous string-like material 70. The heated blades on one hand cut the continuous package material 60 in cross direction and on the other they seal the edges of the cut continuous package material 60. Due to the thermal incompatibility of the continuous package material 60 does not bond to the continuous string-like material 70 upon being fed through the heat cutting section 110, because the melting point of the continuous string-like material 70 is higher than the melting point of the continuous package material 60. Further, the continuous string-like material 70 will not be cut in this step. The cut segment of package material, herein referred to as individual compartments 80, will be displaced versus the non-cut continuous package material 60 in machine direction for exposing the continuous string-like material 70 between the individual compartment 80 and the continuous package material 60.

In the next and final step of the process is forming the drawing means out of the continuous string-like material 70. The individual compartment 80 with the exposed continuous string-like material 70 is fed through a cut & join unit 90. When looked at it in machine direction the cut & join unit 90 joins the two strings of continuous string-like material 70 behind the individual compartment 80 and forms a connection 91 between them. Between the connection 91 and the continuous package material the two strings of continuous string-like material are cut. The joining can be facilitated by any known means, such as stitching, adhesive bonding, heat-bonding, pressure or other mechanical bonding, binding mechanical entanglement knotting or joining by clamps.

This cycle is repeated continuously. The string-like materials forms the drawing means 50 of the container 10. Joining the two string-like materials before the cut at the cut & join unit 90 creates a connection 91 between the two strings. After the connection 91 the two lines of string-like material will be cut. This forms the free ends of the drawing means 50.

All documents cited in the Detailed Description of the Invention are, in relevant part, incorporated herein by reference; the citation of any document is not to be construed as an admission that it is prior art with respect to the present invention. To the extent that any meaning or definition of a term in this written document conflicts with any meaning or definition of the term in a document incorporated by reference, the meaning or definition assigned to the term in this written document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A package comprising:
a container 10 made of flexible material, said container having an interior surface and an exterior surface, said interior surface defming an interior space;
a plurality of absorbent articles disposed within said interior space;
said container having a top end 20 and a bottom end 21,
at or near said top end 20 said container 10 has a channel 42, said channel 42 at least partially enclosing a drawing means 50,
said container 10 further comprises a frangible closure 30 being located at or near said top end 20 and being arranged such that said plurality of absorbent articles is enclosed in said interior space between said bottom end 21 and said closure 30,
**characterized in that** said drawing means 50 is able to move freely inside said channel 42, said drawing means 50 further having two free ends extending outside of the channel 42 for being graspable to the user and wherein said free ends extend outside of said channel 42 at adjacent locations.

2. The package of claim 1, wherein said drawing means 50 is a drawing ribbon or draw string.

3. The package of any of the preceding claims, wherein said frangible closure 30 is selected from perforation lines, weakness lines, tear tapes, and openings in the package covered by a flap.

4. The package of any of the preceding claims, wherein said free ends of said drawing means 50 extending out of the channel 42 are joined to each other outside said channel 42.

5. The package of any of the preceding claims, wherein said top end 20 is provided with a top panel 40 and side extensions 41 extending above the top panel 40, wherein said channel 42 enclosing said drawing means 50 is comprised by said side extensions 41.

6. The package of any of the preceding claims, wherein said two free ends of said drawing means 50 are joined by a frangible joint, which breaks when the user pulls on said free ends.

7. Process for making a container, said process comprising the following steps:
- supplying a continuous package material 60 in a machine direction MD,
- folding said continuous package material 60 such that said continuous package material 60 assumes an M-shaped cross section in a cross-direction, which extends perpendicularly to said machine direction MD, whereby forming tips 61,
- feeding one continuous string-like material 70 in said machine direction into each of the two tips 61 of the M-shaped folded continuous package material 60, said string-like material 70 being thermally incompatible to said package material 60,
- forming channels in said tips 61 by sealing said continuous package material 60 in said machine direction MD near said tips 61, thereby enclosing said continuous string-like material 70 into said channels,
- heat cutting said continuous package material 60 in said cross direction intermittently, whereby individual compartments 80 are formed,
- displacing said individual compartments 80 away from said continuous package material 60 along said machine direction MD,
- feeding said individual compartments 80 through a cut & join unit 90, where said two continuous string-like materials 70 are cut and the ends generated thereby are joined directly adjacent the end of the channels.

8. The process according to claim 7, wherein said ends of said string-like material 70 are joined by at least one of stitching, adhesive bonding, thermo bonding, pressure bonding, binding, mechanical entanglement, knotting or joining by clamps.
